(19) Europäisches Patentamt
European Patent Office
Office européen des brevets

(11) Publication number: **0 112 133**
**B1**

(12) **EUROPEAN PATENT SPECIFICATION**

(45) Date of publication of patent specification: **21.10.87**

(21) Application number: **83307499.0**

(22) Date of filing: **09.12.83**

(51) Int. Cl.[4]: **C 07 D 235/32,**
C 07 D 235/30,
A 61 K 31/415, C 07 C 87/50,
C 07 C 87/58, C 07 C 93/14,
C 07 C 149/32

(54) **5-Phenylethenylbenzimidazoles.**

(30) Priority: **13.12.82 US 449350**

(43) Date of publication of application:
**27.06.84 Bulletin 84/26**

(45) Publication of the grant of the patent:
**21.10.87 Bulletin 87/43**

(84) Designated Contracting States:
**AT BE CH DE FR GB IT LI LU NL SE**

(56) References cited:
**EP-A-0 091 794**
**EP-A-0 091 796**
**AT-B- 358 575**
**CH-A- 627 455**

(73) Proprietor: **SMITHKLINE BECKMAN CORPORATION**
**P.O. Box 7929 1 Franklin Plaza**
**Philadelphia Pennsylvania 19101 (US)**

(72) Inventor: **Chow, Alfred Wen-Jen**
**256 Upper Gulph Road**
**Radnow Pennsylvania 19087 (US)**

(74) Representative: **Waters, David Martin, Dr. et al**
**Smith Kline & French Laboratories Ltd.**
**Patent Department Mundells**
**Welwyn Garden City Hertfordshire AL7 1EY (GB)**

**EP 0 112 133 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to new compounds which have structures characterized by being a 2-acylaminobenzimidazole which is substituted, at the 5-position of its benzene ring, with 1-phenylethenyl. The compounds have anthelmintic activity with a minimum of side effects.

The new compounds of this invention are represented by the following structural formula:

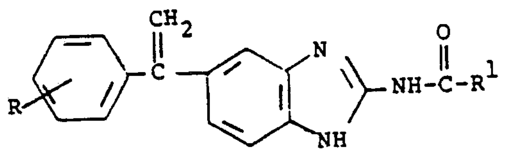

I

in which:

R is hydrogen, halo such as fluoro, chloro or bromo, methoxy, methyl or methylthio.

$R^1$ is lower alkyl of 1—4 carbons or lower alkoxy of 1—4 carbons.

"Lower alkyl" represents straight or branched alkyl groups as well as cycloalkyl groups of 3 or 4 ring members.

A subgroup of the compounds of formula I are those in which R is H or 4-fluoro and $R^1$ is cyclopropyl or methoxy.

The compounds of this invention are prepared by a several step sequence of chemical reactions.

Sequence A

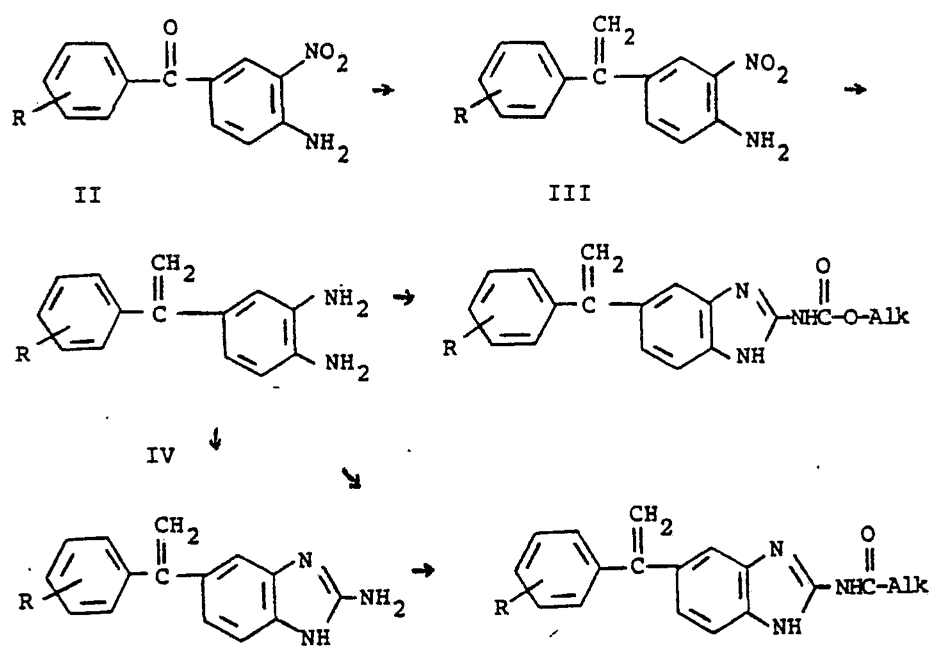

II III IV V

in which R is as defined above and Alk represents a straight, branched or cyclic lower alkyl group of 1—4 carbons which is stable as known to the art.

The first step of the reaction comprises the Wittig reaction which is carried out by reacting a Wittig reagent, such as a ylid, for example, a methylenetriphenylphosphorane, or a compound which forms such a ylid in situ, with an optionally trisubstituted benzophenone (II). The benzophenone starting materials are known in the literature. The reaction is conveniently carried out by reacting (methyl)triphenylphosphonium bromide in the presence of sodium hydride with the selected 3-nitro-4-aminobenzophenone in an inert organic solvent, such as dimethylsulfoxide, at an elevated temperature until the reaction is substantially complete.

Alternatively, the phenylethenyl intermediate is prepared by reacting the benzophenone with a Grignard reagent such as a methyl magnesium halide under standard Grignard conditions to form the α-hydroxyethyl congener which is, then, dehydrated to give the desired ethenyl intermediate usually by heating in the presence of an acid medium.

The resulting 1-phenylethylene compound (III) is then subjected to a mild reducing agent which is

suitable for nitro groups, for example, sodium sulfhydrate in aqueous alcohol, to give the optionally substituted 1-(3,4-diaminophenyl)-1-phenylethylene (IV).

The diamine is cyclized to the end product benzimidazole by any of several reactions known to the art. For example, one reacts cyanamide with the diamine in a one-pot reaction in the presence of an acylating agent, such as a lower alkyl chloroformate or a lower alkanoyl halide such as an alkanoyl chloride or bromide to give the desired product (I). The reaction is, usually, carried out in aqueous alkali-acetone-ethanol in the cold. It will be appreciated that the cyanamide and the acylating agent react first, for example, to form an N-acylcyanamide, for example, carbomethoxycyanamide, which then reacts with the diamine (IV).

As an alternative, the diamine is reacted with a bis-alkoxycarbonyl-S-methylisothiourea or a lower alkyl-S-methyl isothioureacarboxylate, usually in acidic methanol, at reflux. Finally, the 5-(1-phenylethenyl)-2-aminobenzimidazole (V) may be isolated, optionally, from the reaction of the diamine with cyanogen bromide and, then, N-acylated with the noted acylation agents, such as lower alkyl chloroformate or a lower alkanoyl halide or anhydride, usually in the presence of a liquid tertiary organic amine. The latter reaction is particularly useful for preparing the N-alkanoyl end products.

The compounds of structure (III), (IV) and (V) (with the exception of the compound of structure (V) in which R is hydrogen which is disclosed as an intermediate in EP—A—91794 and EP—A—91796) are new and form a further aspect of the invention.

The compounds of this invention have general anthelmintic activity against parasites living in the digestive tract of various mammalian hosts such as humans, swine, cattle, dogs, goats, horses, sheep or cats. Examples of such parasites are the nematodes, such as round worms, hook worms or pinworms, the cestodes, such as the tapeworms, as well as the flukes. The anthelmintic activity is observed following oral administration of a tablet, drench, bolus or other pharmaceutical, animal feed or veterinary composition adapted for oral administration.

For example, the disclosed compounds are generally effective in clearing mice of worm infections for laboratory purposes, among others; Syphacia obvelata and Aspicularis tetraptera (mouse pinworm), Nematospiroides dubius (mouse hookwork) and the migratory stages of Ascaris suum.

Other susceptible helminths include Toxocara canis, found in naturally infested dogs. Also, parasitic to this host are Ancylostoma canium, Trichuris vulpis (whipworm) and Physalaptera ssp.

These compounds are efficacious against parasites of pigs, such as the migratory stages of Ascaris suum, thus preventing the development of verminous pneumonia.

Among the gastrointestinal parasites in sheep and cattle which are susceptible are Haemonchus contortus, Ostertagia ssp., Trichostrongylus ssp., Nematodirus spp., Trichuris ovis, Cooperia spp., Strongyloides papillosus, Bunostomum spp., Chabertia sp. and Oesophagostomum spp.

In practice, the active 5-(1-phenylethenyl)-benzimidazole compound is, usually, formulated with a nontoxic pharmaceutical, veterinary or feed carrier therefor to give the anthelmintic compositions of this invention. The carrier is a standard animal feed composition, which is based on a feed premix or a whole feed composition, or an orally ingestible anthelmintic carrier for the active ingredient in a drench, a dispersible tablet or powder, or a bolus form. It may also be combined with a pharmaceutically acceptable diluent or excipient of the kind normally used in the production of veterinary or human medicaments, for example, maize, starch, terra alba, lactose, sucrose, calcium phosphate, gelatin, talcum, stearic acid, magnesium stearate, dextrin, agar, pectin or acacia.

Exemplary of liquid carriers are peanut oil, olive oil, sesame oil and water. Similarly, the carrier or diluent may include a time delay material, such as glyceryl monostearate or glyceryl distearate alone or with a wax.

The compositions are advantageously made up in a dosage unit form adapted for the desired frequency and mode of administration. Thus, for the preferred oral administration, the dosage unit may take the form of a suspension, top dressing, tablet, packaged powder, bolus or encapsulated dispensible powder. The quantity of active ingredient in each dosage will be such that one or more units are required for each therapeutic administration.

Where tableting is used, the resulting tablets may then be coated with methyl methacrylate to form an enteric coating, i.e., a coating which is substantially insoluble in gastric secretion but substantially soluble in intestinal fluids.

The compositions, thusly prepared using a anthelmintic, but nontoxic, quantity chosen from the range of 2—15 mg/kg., are administered, usually orally, to an infected or susceptible host from 1—5 times daily for curative or prophylatic anthelmintic activity. Preferably, one dose daily is used but often doses in the field may be singular or repeated for 1—3 times at monthly intervals. The most important species of this invention is methyl [5-(1-phenylethenyl)-1H-benzimidazol-2-yl]-carbamate which has a low level of embryotoxicity compared with standard prior art benzimidazole anthelmintics such as parbendazole.

Exemplary of the anthelmintic activity of this new series of compounds are the following results[a]:

A. Compound of Example 3 —
Methyl[5-(1-phenylethenyl)-1H-benzimidazol-2-yl]carbamate

| Nematospiroides dubius-Mouse | Fasciola Hepatica-Mouse[b] | Fasciola Hepatica-Sheep[c] |
|---|---|---|
| 86% at 0.006% | 83/19 at 0.05% | 95% at 5 mg/kg |
| 31% at 0.05% | 50/0 at 0.05% | |
| 0% at 0.025% | | |
| 51% at 0.1% | | |

B. Compound of Example 4 —
N-[5-(1-phenylethenyl)-1H-benzimidazol-2-yl]cyclopropanecarboxamide

| | | |
|---|---|---|
| 14% at 0.025% | 67/0 at 0.025% | |

[a]Determined by the methods outlined in V. J. Theodorides "Anthelmintics: From Laboratory Animals to the Target Species" Chapter 5, Chemotherapy of Infectious Diseases, pages 71—93, Ed. H. H. Gadebusch, C.R.C. Press, 1976.

[b]Percent by weight of diet. The first figure is an index of hepatic pathology, the second is percent reduction of worm burden.

[c]Milligrams of compound per kilogram of body weight. In addition, the first compound, A, was found not to be embryotoxic, a serious side effect which many anthelmintic benzimidazoles possess.

The following examples illustrate specific aspects of the invention which may be employed in preparing and using the compositions of the invention but are not intended to limit the scope of the invention described hereinbefore. Degrees of temperature are in Centigrade unless otherwise noted.

## Example 1

| Typical Cattle Bolus | |
|---|---|
| Methyl [5-(1-phenylethenyl)-1H-benzimidazole-2-yl]carbamate | 0.20 grams |
| Calcium Phosphate | 2.5 grams |
| Maize Starch | 0.54 grams |
| Talcum | 0.14 grams |
| Gum Arabic | 0.15 grams |
| Magnesium Stearate | 0.5 grams |

The calcium phosphate and the anthelmintic compound are thoroughly mixed, and the mixture reduced to a particle size finer than 60 mesh. About one-half of the starch is added, as an aqueous paste, and the resulting mixture granulated. The granules are passed through a 10 mesh screen and dried at 110°—130°F for about eight hours. The dried materials are then passed through a No. 16 mesh screen. The guar gum and the balance of the starch are added and the mixture thoroughly blended. Finally, the remainder of the ingredients are added and the entire mass thoroughly mixed and compressed into a bolus. The magnesium stearate, talcum and gum acacia are of a particle size to pass a No. 10 mesh screen.

Example 2

| Sheep Drench | Parts by Weight |
|---|---|
| Methyl [5-(1-phenylethenyl)-1H-benzimidazol-2-yl]carbamate | 20 |
| Terra Alba English | 65.5 |
| Tragacanth, U.S.P. | 3.0 |
| Sodium Lauryl Sulfate | 1.5 |
| Water | |

The above solid components are thoroughly mixed, giving a water dispersible powder. This powder can be directly admixed with water in concentrations on the order of 10.5 g. of powder to 5 cc. of water.

Example 3

Sodium hydride (2.4 g., 0.1 mole) was suspended in dimethyl sulfoxide (50 ml.) and heated at 70° under nitrogen until evolution of hydrogen gas ceased (approximately ½ hour). To this suspension at room temperature was added a solution of (methyl)triphenylphosphonium bromide (35.7 g., 0.1 mole) in dimethyl sulfoxide (100 ml.). The greenish cloudy solution was stirred at ambient temperature for fifteen minutes. To this was, then, added (4-amino-3-nitrophenyl)phenylmethanone (12.1 g., 0.05 mole). The resulting dark red solution was heated at 90° for 18 hours under nitrogen. The reaction mixture was, then, cooled and diluted with water (500 ml.). The pH was adjusted to 7.0 by the addition of 3N hydrochloric acid. This solution was, then, extracted with ethyl ether (2 × 150 ml.). The combined organic extracts were washed with water (500 ml.), dried over sodium sulfate and evaporated to dryness. The resulting dark oil was chromatographed over silica gel, yielding an orange solid; 1-(4-amino-3-nitrophenyl)-1-phenylethylene: 7.7 g., 64% yield, m.p. 136—7°.

1-(4-Amino-3-nitrophenyl)-1-phenylethylene (7.7 g., 0.032 mole) was dissolved in refluxing ethanol (450 ml.). To this was added, dropwise, a solution of sodium sulfhydrate (38.5 g., 0.45 mole) in water (110 ml.). The resulting solution was refluxed overnight. The solution was diluted with water (1 l.), cooled, and filtered yielding 1-(3,4-diaminophenyl)-1-phenylethylene: 5.6 g., 83% yield, m.p. 109—110°.

1-(3,4-Diaminophenyl)-1-phenylethylene (3.2 g., 0.015 mole) was dissolved in methanol (100 ml.). To this was added water (32 ml.), 1,3-bis(methoxycarbonyl)-S-methylisothiourea (3.2 g., 0.015 mole), and acetic acid (3 drops). The reaction solution was, then, refluxed for 1.5 hours, cooled and the product collected by filtration. This tan solid was dissolved in warm methanol (100 ml.) and 3N hydrochloric acid (50 ml.). The solution was cooled, filtered, and, then, neutralized to pH 7.0 by a 5% sodium bicarbonate solution. The precipitate was collected by filtration, yielding methyl [5-(1-phenylethenyl)-H-benzimidazol-2-yl]carbamate: 4.0 g., 91% yield, m.p. 217—8°.

Anal. Calcd. for $C_{17}H_{15}N_3O_2$, C, 69.61; H, 5.15; N, 14.33; Found: C, 69.46; H, 5.20; N, 14.11.

Example 4

A mixture of 3.6 g. (15 mmole) of 2-amino-5-(1-phenylethenyl)-benzimidazole [prepared from the diamine described in Example 3 by using cyanogen bromide as described by J. H. Wikel, et al., J. Med. Chem. *23* 368 (1980)], 3.12 g. (30 mmole) of cyclopropane carboxylic acid chloride and 100 ml. of pyridine was allowed to react at room temperature, quenched and the product isolated to give 3.4 g. (75%) of N-[5-(1-phenylethenyl)-1H-benzimidazol-2-yl]cyclopropanecarboxamide, hydrate, m.p. 199—202°.

Anal. Calcd. for $C_{19}H_{17}N_3O \cdot 1/8\ H_2O$: C, 74.67; H, 5.68; N, 13.75. Found: C, 74.47; H, 5.72; N, 14.03.

Using acetyl chloride gives the corresponding acetamide, using propionyl chloride gives the corresponding propionamide and using propylchloroformate gives propyl [5-(1-phenylethenyl)-1H-benzimidazol-2-yl]carbamate.

Example 5

To a stirring solution of 19.9 g (0.1 mole) of 3-nitro-4-aminoacetophenone (Annalen *553,* 250—9, 1942; CA *37* 5044, 1943) in 1 l of anhydrous ether, under a nitrogen atmosphere, was slowly added an ethereal solution of *p*-fluorophenyl magnesium bromide (0.3 mole). After the addition, the reaction mixture was refluxed for two hours. To this reaction mixture was added, cautiously, 100 ml of saturated ammonium chloride solution. After stirring for 0.5 hours, the ethereal solution was separated and concentrated to give the tertiary carbinol as an oil. This was suspended in 240 ml of 3N hydrochloric acid, heated with stirring at 100° for 1 hour and cooled. The residue was taken up in a small volume of ethanol. Addition of ether gave 1-(4-amino-3-nitrophenyl)-1-(4-fluorophenyl)-ethylene as the hydrochloride salt, 13.5 g (46%).

To a refluxing solution of 12.9 g (0.05 mole) of the hydrochloride in a mixture of 500 ml of ethanol and

150 ml of water was added slowly a solution of 40 g of sodium sulfhydrate ($NaSH \cdot XH_2O$) in 50 ml of water. The resulting mixture was refluxed for five hours, at which time, thin layer analysis (silica gel, 1:1 ether/n-hexane) demonstrated the absence of the starting material and the appearance of the diamine. The reaction mixture was poured into 3 l of water and the solid which separated was removed by filtration, dissolved in 300 ml of methylene chloride and extracted with 2 × 500 ml of 1N hydrochloric acid. The hydrochloric acid extract was neutralized with 40% sodium hydroxide solution and the solid was filtered off to give 9.6 g (85%) of the diamine, m.p. 107—108°. This was treated with the theoretical amount of conc. hydrochloric acid to give the dihydrochloride salt.

To a stirring solution of 10 g (0.12 mole of a 50% (w/w) aqueous cyanamide solution diluted with 10 ml of water and 25 ml of acetone was added 12.3 g (0.13 mole) of methylchloroformate. The resulting mixture was cooled to a −30° with a dry ice-acetone bath, and 12.5 ml of a 50% aqueous sodium hydroxide solution diluted with an equal volume of water was added slowly keeping the temperature of the reaction at −20° to −30°. The final solution is about pH 6—7. This is prepared in situ and used directly in the next step.

The diamine dihydrochloride prepared above was suspended in 300 ml of water and heated to 60°. With rapid stirring, the carbmethoxy cyanamide solution prepared above was added as one portion. The resulting mixture was heated at 80—90° for three hours and the resulting solid filtered, washed with water, methanol and acetone and dried to give 9.1 g of methyl [5-(1-4-fluorophenylethenyl)-1H-benzimidazol-2-yl]carbamate as an off white solid, m.p. 221—222°.

Methyl [5-(1-phenylethenyl)-1H-benzimidazol-2-yl]-carbamate is prepared similarly.

Example 6

Substituting (4-amino-3-nitrophenyl)-3-methylphenylmethanone, (4-amino-3-nitrophenyl)-4-methoxyphenylmethanone, (4-amino-3-nitrophenyl)-4-bromophenylmethanone or (4-amino-3-nitrophenyl)-2-chlorophenylmethanone in the method of Example 3 gives methyl 5-[1-(3-methylphenyl)ethenyl]-1H-benzimidazol-2-yl carbamate, methyl 5-[1-(4-methoxyphenyl)ethenyl]-1H-benzimidazol-2-yl carbamate, methyl 5-[1-(4-bromophenyl)-ethenyl]-1H-benzimidazol-2-yl carbamate and methyl 5-[1-(2-chlorophenyl)ethenyl]-1H-benzimidazol-2-yl carbamate respectively.

**Claims for the Contracting States: BE CH DE FR GB IT LI LU NL SE**

1. A chemical compound of the formula:

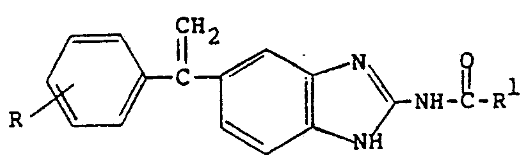

in which

R is hydrogen, halo, methyl, methoxy or methylthio; and

$R^1$ is lower alkyl of 1—4 carbons, cycloalkyl of 3 or 4 carbon atoms or lower alkoxy of 1—4 carbons.

2. The compound of Claim 1 in which R is hydrogen or 4-fluoro and $R^1$ is methoxy.

3. The compound of Claim 1 being methyl [5-(1-phenylethenyl)-1H-benzimidazol-2-yl)carbamate.

4. The compound of Claim 1 being methyl 5-[1-(4-fluorophenyl)-ethenyl]-1H-benzimidazol-2-yl] carbamate.

5. The compound of Claim 1 being N-[5-(1-phenylethenyl)-1H-benzimidazol-2-yl]-cyclopropanecarboxamide.

6. A compound of one of Claims 1—5 for use as an anthelmintic.

7. The method of preparing a compound of structural formula:

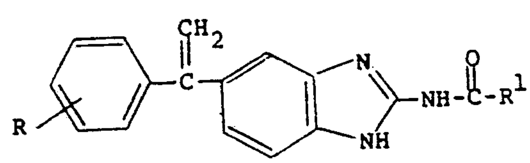

in which:

R is hydrogen, halo, methyl, methoxy or methylthio; and

$R^1$ is lower alkyl of 1—4 carbons, cycloalkyl of 3 or 4 carbon atoms or lower alkoxy of 1—4 carbons, comprising reacting a diamino compound of the structural formula

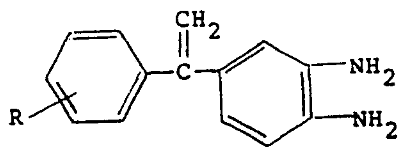

in which R is as defined above, with either:

(A) cyanamide and a lower alkyl chloroformate or a lower alkanoyl halide,

(B) a N-lower alkoxycarbonyl-S-methylisothiourea, or a bis(alkoxycarbonyl)-S-methylisothiourea, or

(C) cyanogen bromide and, then, in a separate step, a lower alkanoyl halide or a lower alkyl chloroformate.

8. The method of Claim 7 in which the reaction is used to prepare a compound in which $R^1$ is lower alkoxy and the diamine is reacted with cyanamide and a lower alkyl chloroformate.

9. A compound of the formula,

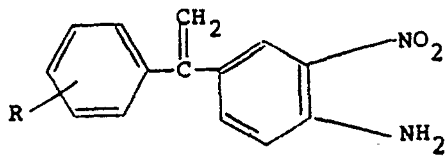

in which R is hydrogen, halo, methyl, methoxy or methylthio.

10. A compound of the formula

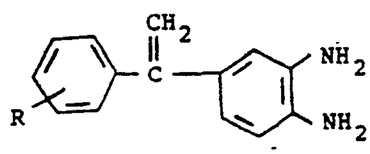

in which R is hydrogen, halo, methyl, methoxy or methylthio.

11. A compound of the formula

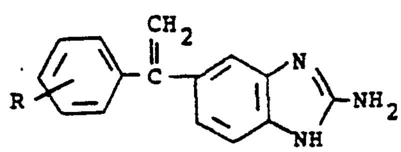

in which R is halo, methyl, methoxy or methylthio.

12. An anthelmintic composition comprising a compound of claim 1 in association with a pharmaceutical, veterinary or feed carrier therefor.

13. An anthelmintic composition as claimed in claim 12 in unit dosage form for oral administration wherein the amount of compound of claim 1 is from 2—15 mg/kg bodyweight of the recipient thereof.

**Claims for the Contracting State: AT**

1. The method of preparing a compound of the structural formula:

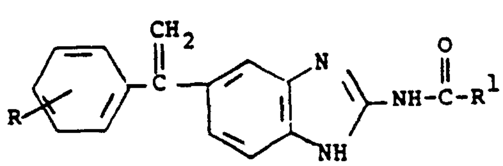

in which:

R is hydrogen, halo, methyl, methoxy or methylthio; and

$R^1$ is lower alkyl of 1—4 carbons, cycloalkyl of 3 or 4 carbon atoms or lower alkoxy of 1—4 carbons, comprising reacting a diamino compound of the structural formula:

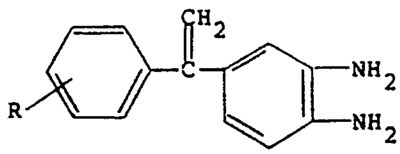

in which R is as defined above, with either:

(A) cyanamide and a lower alkyl chloroformate or a lower alkanoyl halide,

(B) a N-lower alkoxycarbonyl-S-methylisothiourea or a bis(alkoxycarbonyl)-S-methylisothiourea, or

(C) cyanogen bromide and, then, in a separate step, a lower alkanoyl halide or a lower alkyl chloroformate.

2. The method of claim 1 in which the reaction is used to prepare a compound in which $R^1$ is lower alkoxy and the diamine is reacted with cyanamide and a lower alkyl chloroformate.

3. The method of claim 1 in which R is hydrogen and $R^1$ is methoxy and the diamino compound is reacted with cyanamide and methyl chloroformate.

4. The method of claim 1 in which R is hydrogen and $R^1$ is methoxy and the diamino compound is reacted with bis(methoxycarbonyl)-S-methylisothiourea.

5. The method of claim 1 in which R is hydrogen and $R^1$ is cyclopropyl and the diamino compound is reacted first with cyanogen bromide and, then, with cyclopropanecarboxylic acid chloride.

**Patentansprüche für die Vertragsstaaten: BE CH DE FR GB IT LI LU NL SE**

1. Eine chemische Verbindung der Formel

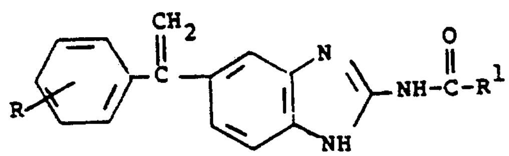

in der

R ein Wasserstoff- oder Halogenatom, eine Methyl- oder Methoxy- oder Methylthiogruppe; und

$R^1$ einen Niederalkylrest mit 1 bis 4 Kohlenstoffatomen, einen Cycloalkylrest mit 3 oder 4 Kohlenstoffatomen oder einen Niederalkoxyrest mit 1 bis 4 Kohlenstoffatomen bedeuten.

2. Die Verbindung nach Anspruch 1, wobei R ein Wasserstoffatom oder ein 4-Fluoratom und $R^1$ eine Methoxygruppe bedeuten.

3. Die Verbindung nach Anspruch 1, nämlich Methyl-[5-(1-phenyläthenyl)-1H-benzimidazol-2-yl)-carbamat.

4. Die Verbindung nach Anspruch 1, nämlich Methyl-5-[1-(4-fluorphenyl)-äthenyl]-1H-benzimidazol-2-yl]carbamat.

5. Die Verbindung nach Anspruch 1, nämlich N-[5-(1-Phenyläthenyl)-1H-benzimidazol-2-yl]-cyclopropan-carboxamid.

6. Eine Verbindung nach einem der Ansprüche 1 bis 5 zur Verwendung also Anthelmintikum.

7. Verfahren zur Herstellung einer Verbindung der Strukturformel:

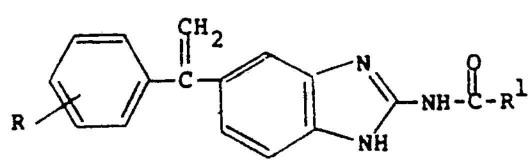

in der:

R ein Wasserstoff- oder Halogenatom, eine Methyl-, Methoxy-, oder Methylthiogruppe; und

$R^1$ einen Niederalkylrest mit 1 bis 4 Kohlenstoffatomen, einen Cycloalkylrest mit 3 bis 4 Kohlenstoffatomen oder einen Niederalkoxyrest mit 1 bis 4 Kohlenstoffatomen bedeuten, durch Umsetzung einer Diaminoverbindung der Strukturformel

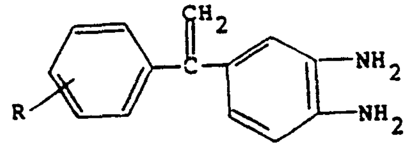

in der R die vorstehende Bedeutung hat, mit entweder:

(A) Cyanamid und einem Niederalkylchlorformat oder einem Niederalkanoylhalogenid,

(B) einem N-Niederalkoxycarbonyl-S-methylisothioharnstoff oder einem Bis(alkoxycarbonyl)-S-methylisothioharnstoff, oder

(C) Bromcyan und, anschließend, in einer getrennten Stufe, einem Niederalkanoylhalogenid oder einem Niederalkylchlorformat.

8. Verfahren nach Anspruch 7, wobei die Umsetzung zur Herstellung einer Verbindung erfolgt, in der $R^1$ einen Niederalkoxyrest bedeutet, und das Diamin mit Cyanamid und einem Niederalkylchlorformat umgesetzt wird.

9. Eine Verbindung der Formel

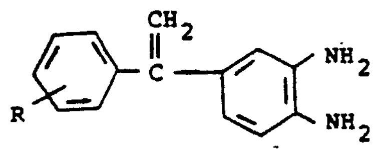

in der R ein Wasserstoff- oder Halogenatom, eine Methyl-, Methoxy- oder Methylthiogruppe bedeutet.

10. Eine Verbindung der Formel

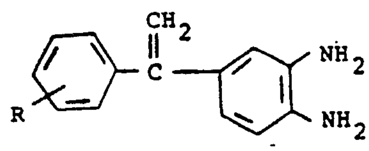

in der R ein Wasserstoff- oder Halogenatom oder eine Methyl-, Methoxy- oder Methylthiogruppe bedeutet.

11. Eine Verbindung der Formel

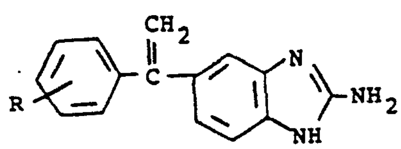

in der R ein Halogenatom oder eine Methyl-, Methoxy- oder Methylthiogruppe bedeutet.

12. Eine anthelmintische Zusammensetzung, enthaltend eine Verbindung nach Anspruch 1 in Verbindung mit einem pharmazeutischen, veterinärmedizinischen oder Futtermittelträgerstoff.

13. Eine anthelmintischen Zusammensetzung nach Anspruch 12 in Form einer Einzeldosis zur oralen Applikation, in der die Menge der Verbindung nach Anspruch 1 bei 2 bis 15 mg/kg Körpergewicht des Empfängers liegt.

**Patentansprüche für den Vertragsstaat: AT**

1. Verfahren zur Herstellung einer Verbindung der Strukturformel:

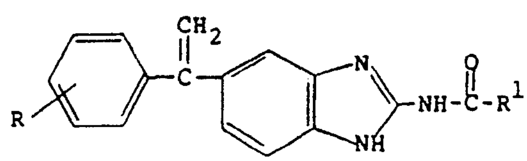

in der:

R ein Wasserstoff- oder Halogenatom, eine Methyl-, Methoxy-, oder Methylthiogruppe; und

$R^1$ einen Niederalkylrest mit 1 bis 4 Kohlenstoffatomen, einen Cycloalkylrest mit 3 bis 4 Kohlenstoffatomen oder einen Niederalkoxyrest mit 1 bis 4 Kohlenstoffatomen bedeuten,

durch Umsetzung einer Diaminoverbindung der Strukturformel

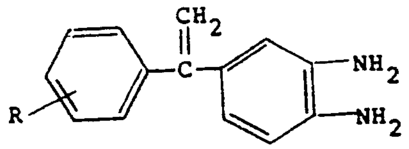

in der R die vorstehende Bedeutung hat, mit entweder:

(A) Cyanamid und einem Niederalkylchlorformat oder einem Niederalkanoylhalogenid,

(B) einem N-Niederalkoxycarbonyl-S-methyliso thioharnstoff oder einem Bis(alkoxycarbonyl)-S-methylisothioharnstoff, oder

(C) Bromcyan und, anschließend, in einer getrennten Stufe, einem Niederalkanoylhalogenid oder einem Niederalkylchlorformat.

2. Verfahren nach Anspruch 1, wobei die Umsetzung zur Herstellung einer Verbindung erfolgt, in der $R^1$ einen Niederalkoxyrest bedeutet und das Diamin mit Cyanamid und einem Niederalkylchlorformat umgesetzt wird.

3. Verfahren nach Anspruch 1, wobei R ein Wasserstoffatom und $R^1$ eine Methoxygruppe bedeuten und die Diaminoverbindung mit Cyanamid und Methylchlorformat umgesetzt wird.

4. Verfahren nach Anspruch 1, wobei R ein Wasserstoffatom und $R^1$ eine Methoxygruppe bedeuten und die Diaminverbindung mit Bis-(methoxycarbonyl)-S-methylisothioharnstoff umgesetzt wird.

5. Verfahren nach Anspruch 1, wobei R ein Wasserstoffatom und $R^1$ eine Cyclopropylgruppe bedeutet und die Diaminoverbindung zunächst mit Bromcyan und anschließend mit Cyclopropancarbonsäurechlorid umgesetzt wird.

**Revendications pour les Etats contractants: BE CH DE FR GB IT LI LU NL SE**

1. Un composé chimique de la formule:

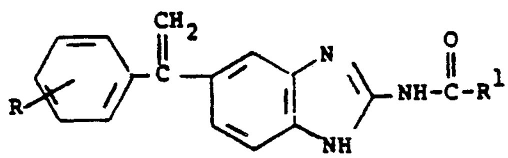

dans laquelle:

R est hydrogène, halo, méthyle, méthoxy ou méthylthio; et

$R^1$ est alcoyle plus faible de 1—4 carbones, cycloalcoyle de 3 ou 4 atomes de carbone ou bien alcoxy plus faible de 1—4 carbones.

2. Le composé de la Revendication 1 dans laquelle R est hydrogène ou 4-fluoro et $R^1$ est méthoxy.

3. Le composé de la Revendication 1 étant du méthyle [5-(1-phényéthenyl)-1H-benzimidazol-2-yl)carbamate.

4. Le composé de la Revendication 1 étant du méthyle 5-[1-(4-fluorophényle)-éthenyl]-1H-benzimidazol-2-yl] carbamate.

5. Le composé de la Revendication 1 étant N-[5-(1-phényléthenyl]-1H-benzimidazol-2-yl]-cyclopropanecarboxamide.

6. Un composé de l'une des Revendications de 1—5 à employer comme anthelmintique.

7. La méthode de préparation d'un composé de la formule structurelle:

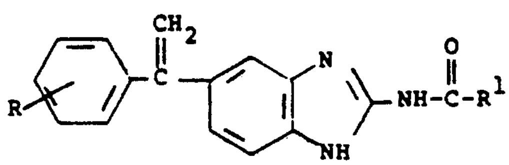

dans laquelle

R est hydrogène, halo, méthyle, méthoxy ou méthylthio; et

$R^1$ est alcoyle plus faible de 1—4 carbones, cycloalcoyle de 3 ou 4 atomes de carbone ou alcoxy plus faible de 1—4 carbones,

comprenant la mise en réaction d'un composé diamino de la formule structurelle

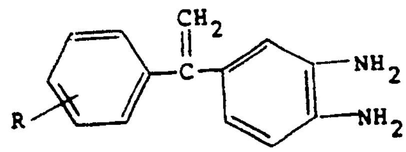

dans laquelle R est comme défini ci-dessus, avec soit:

(A) cyanamide et un chloroformate d'alcoyle plus faible ou bien un halogénure d'alcanoyl plus faible,

(B) un N-plus faible alcoxycarbonyle-S-méthylisothiourée, ou un bis(alcoxycarbonyle)-S-méthylisothiourée, ou

(C) du bromure de cyanogène et, ensuite, dans un phase séparée, un halogénure d'alcanoyl plus faible ou bien un chloroformate d'alcoyle plus faible.

8. La méthode de la Revendication 7 dans laquelle la réaction sert à préparer un composé dans lequel $R^1$ est de l'alcoxy plus faible et la diamine est mise en réaction avec de la cyanamide et un chloroformate d'alcoyle plus faible.

9. Un composé de la formule,

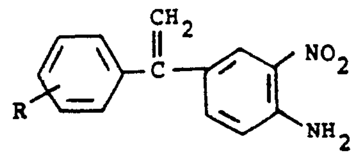

dans laquelle R est hydrogène, halo, méthyle, méthoxy ou méthylthio.

10. Un composé de la formule

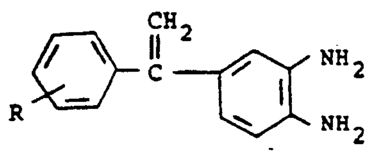

dans laquelle R est hydrogène, halo, méthyle, méthoxy ou méthylthio.

11. Un composé de la formule

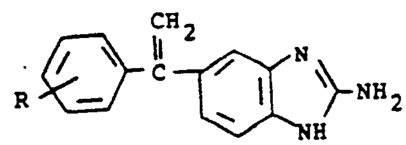

dans laquelle R est halo, méthyle, méthoxy ou méthylthio.

12. Une composition anthelmintique comprenant un composé de la Revendication 1 en association avec un support pharmaceutique, vétérinaire ou alimentaire du même.

13. Une composition anthelmintique comme indiqué dans la revendication 12 sous forme de dosage unitaire pour administration orale dans laquelle la quantité du composé de la revendication 1 est entre 2 et 15 mg/kg de poids du corps du receveur du même.

**Revendications pour l'Etat contractant: AT**

1. La méthode de préparation d'un composé de la formule structurelle:

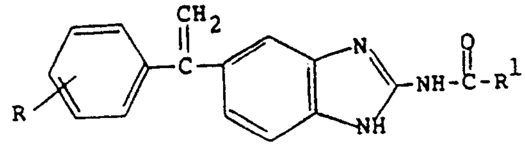

dans laquelle

R est hydrogène, halo, méthyle, méthoxy ou méthylthio; et

$R^1$ est alcoyle plus faible de 1—4 carbones, cycloalcoyle de 3 ou 4 atomes de carbones our alcoxy plus faible de 1—4 carbones, comprenant la mise en réaction d'un composé diamino de la formule structurelle:

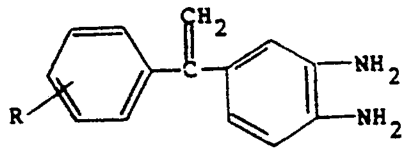

dans laquelle R est comme défini ci-dessus, avec soit:

(A) de la cyanamide et du chloroformate d'alcoyle plus faible ou bien un halogénure d'alcanoyl plus faible,

(B) un N-plus faible alcoxycarbonyle-S-méthylisothiourée ou bien un bis(alcoxycarbonyle)-S-méthylisothiourée, ou bien

(C) du bromure de cyanogène et, ensuite, en une phase séparée, un halogénure d'alcanoyl plus faible ou bien un chloroformate d'alcoyl plus faible.

2. La méthode de la revendication 1 dans laquelle la réaction sert à préparer un composé dans lequel $R^1$ est alcoxy plus faible et la diamine est mise en réaction avec de la cyanamide et un chloroformate d'alcoyl plus faible.

3. La méthode de la revendication 1 dans laquelle R est hydrogène et $R^1$ est méthoxy et le composé diamino est mis en réaction avec de la cyanamide et du chloroformate de méthyle.

4. La méthode de la revendication 1 dans laquelle R est hydrogène et $R^1$ est méthoxy et le composé diamino est mis en réaction avec bis(méthoxycarbonyle)-S-méthylisothiourée.

5. La méthode de la revendication 1 dans laquelle R est hydrogène et $R^1$ est cyclopropyl et le composé diamino est mis en réaction d'abord avec du bromure de cyanogène et, ensuite, avec du chlorure d'acide cyclopropanecarboxylique.